# EUROPEAN PATENT APPLICATION

(11) **EP 0 741 141 A2**
(43) Date of publication of application: **06.11.1996**
(21) Application number: 95115478.0
(22) Date of filing: 29.09.1995
(51) Int. Cl.: C07H 21/00

(54) **Method of purifying ologonucleotide from biological samples**

(30) Priority: 04.05.1995 US 19950434111
(71) Applicant: Hewlett-Packard Company, Palo Alto, California 94304 (US)
(72) Inventor: Lichtenwalter, Kay, San Jose, California 95127 (US)
(74) Representative: Schoppe, Fritz, Dipl.-Ing.

(57) **Abstract**

Methods are provided for purifying oligonucleotides from biological samples using magnetically responsive particles. The method involves incubating magnetically responsive particles, to which oligonucleotide-binding partners have been linked, with biological sample preparations to form a particle-binding partner-analyte complex. The complex is then separated from the cellular debris by application of a magnetic field.

## Description

### TECHNICAL FIELD

This invention relates generally to methods of purifying cellular constituents. More particularly, the invention relates to methods for purifying oligonucleotides from biological samples using magnetically responsive particles to which oligonucleotide-binding partners have been linked.

### BACKGROUND

Clinical application of molecular biology and recombinant DNA technology has greatly advanced the understanding of human hereditary disorders. To exploit the benefits of these advances, it is essential to have methods for rapidly and easily separating, isolating and purifying high quality, intact oligonucleotides.

One of the earliest described methods for purifying oligonucleotides involved precipitation of bacterial DNA with alkyltrimethylammonium bromide. Jones (1953) Biochim. Biophys. Acta 10:607-612. A subsequent series of extractions and precipitations which are required to obtain purified DNA takes one to two days.

Marmur (1961) J. Mol. Biol. 3:208-218 subsequently described an equally lengthy procedure for isolating DNA which also involves a series of extractions and precipitations using phenol and chloroform. A typical protocol derived from the original Marmur procedure involves an initial overnight incubation of a sample at 55° in sodium chloride-Tris-EDTA (STE) buffer with proteinase K or pronase to digest protein present in the sample. The digested proteins are removed from the sample extraction with phenol/chloroform. The aqueous phase containing oligonucleotides is recovered and dialyzed overnight against buffer. Oligonucleotides are recovered from the sample by precipitation with ethanol or isopropanol. RNA is removed from the ethanol precipitate by treatment with RNase followed by a second phenol/chloroform extraction and a second overnight dialysis. The concentration of DNA in the final dialysate is determined by taking the ratio of the absorbance at 260 nm to that at 280 nm.

This procedure is still considered to be effective in removing proteins and quantitatively recovering oligonucleotide; however, this method has certain deficiencies which limits its use in a clinical laboratory setting. First, the isolation method takes one to two days. Second, the phenol/chloroform extraction procedure may result in the isolation of a suboptimal, and even unacceptable, oligonucleotide product. For example, to completely remove proteins, the chloroform/phenol extraction must be repeated several times. Oligonucleotides can be damaged by oxidation products of phenol, and this repetitive extraction process is subject to loss of product with each chloroform/phenol extraction. Third, the yield of DNA from this method is poor and large quantities of source material is required. Finally, the use of hazardous organic solvents makes such a procedure unacceptable for clinical applications.

Other methods of isolating oligonucleotides have been developed to avoid the use of caustic organic chemicals and lengthy sample processing time. However, problems still exist with these methods. Some require a highly purified source material, while others are only effective in isolating small genomic DNA from, for example, bacteria. Other methods do not lend themselves readily to rapid batch processing of large numbers of samples.

Because of these limitations in easily obtaining highly purified oligonucleotides, it has not yet become common practice to isolate oligonucleotides for diagnostic hybridization or other assays in the clinical laboratory. A method which would be useful for extracting oligonucleotides from biological samples for use in clinical assays must be: 1) rapid, taking on the order of minutes to complete; 2) easy to perform, requiring minimal sample preparation; 3) free of harsh, hazardous organic chemicals; 4) sufficiently flexible to extract DNA or RNA, single or double stranded, any length; and 5) produce highly purified oligonucleotide.

### RELATED ART

Other methods by which oligonucleotides may be isolated from biological samples have been reported and are exemplified by the following:
U.S. Patent No. 4,908,318, issued 13 March 1990 to Lerner, describes a method of extracting oligonucleotides from a buffy coat fraction of a blood sample by lysing the cells present therein, solubilizing and then selectively precipitating the oligonucleotides consequently released.
U.S. Patent No. 4,935,342, issued 19 June 1990 to Seligson et al., describes a method of isolating and purifying oligonucleotides from biological samples using anion exchange column chromatography.
U.S. Patent No. 4,983,523, issued 8 January 1991 to Li et al., describes a method of preparing oligonucleotides for hybridization by lysing cells using non-invasive sonication of a sample.
International Publication No. WO 93/03150 describes a method of preparing single strand oligonucleotide shear fragments for hybridization using non-invasive sonication of cells in a chaotropic agent, shearing oligonucleotides thereby released into uniform-length fragments and denaturing the fragments into single-stranded oligonucleotides.
Verma (1988) BioFeedback 6:849-851 describes a method of isolating DNA suitable for RFLP analysis using high concentrations of chaotropic agents.
Yanamandra et al. (1989) Gene Anal. Techn. 6:71-74 describe a method of isolating and characterizing DNA using an agarose gel method which entails suspending cells in an agarose solution, solidifying the agarose solution and processing the cells in the agarose gel using conventional methods thereby yielding higher molecular weight DNA compared with DNA isolated using conventional techniques. The processing takes at least a day.
Majumdar et al. (1991) BioTechniques 11:940-101 describes a method of isolating DNA by lysing cells with a detergent, extracting the oligonucleotide from the aqueous sample with phenol:chloroform:isoamyl alcohol and eluting the DNA from the organic phase by increasing the pH.

### SUMMARY OF THE INVENTION

To address the above-mentioned need in the art, the invention disclosed and claimed herein provides a method for preparing highly purified oligonucleotides which are adequate for clinical assays and other assays which require oligonucleotides purified to remove contaminating substances. The method employs a magnetically responsive particle which is surface-coated with an oligonucleotide-binding partner. The coated particle is added to a biological sample. Oligonucleotides bind to the particle by virtue of their interaction with the binding partner and are then separated from the sample by application of a magnetic field.

Accordingly, it is an object of the invention to provide a method of purifying a target oligonucleotide analyte by providing a sample containing the target analyte, contacting the sample with a magnetically responsive particle to which an oligonucleotide analyte-binding partner has been linked, incubating the sample with the particle, thereby forming a particle-binding partner-analyte complex, exposing the sample to a magnetic field, and separating the complex from the sample.

### DETAILED DESCRIPTION OF THE INVENTION

The practice of the present invention will employ, unless otherwise indicated, conventional techniques of molecular biology, microbiology, recombinant DNA technology, and immunology, which are within the skill of the art. Such techniques are explained fully in the literature. *See, e.g.,* Sambrook, Fritsch & Maniatis, Molecular Cloning: A Laboratory Manual, Second Edition (1989); Oligonucleotide Synthesis (M.J. Gait ed. 1984); Immobilized Cells and Enzymes (IRL press, 1986); the series, Methods In Enzymology (S. Colowick and N. Kaplan eds., Academic Press, Inc.); and Handbook of Experimental Immunology, Vols. I-IV (D.M. Weir and C.C. Blackwell eds., 1986, Blackwell Scientific Publications).

### A. Definitions:

Before the invention is described in detail, it is to be understood that this invention is not limited to specific analyte-binding partners, magnetically responsive particles or coating techniques as such may vary. It is also to be understood that the terminology used herein is for the purpose of describing particular embodiments only and is not intended to be limiting. It must be noted that, as used in the specification and the appended claims, the singular forms "a," "an" and "the" include plural referents unless the context clearly dictates otherwise. Thus, for example, reference to "an analyte" includes mixtures of analytes, reference to "an analyte-binding partner" includes mixtures of two or more such binding partners, and the like. In this regard, it is important to note that the techniques of the present invention may be used to purify multiple analytes from a sample, e.g., as captured on a magnetically responsive particle.

In this specification and in the claims which follow, reference will be made to a number of terms which shall be defined to have the following meanings:

As used herein, the term "oligonucleotide" include double- and single-stranded DNA, as well as double- and single-stranded RNA. The term "oligonucleotide analyte" refers to a single- or double-stranded oligonucleotide to which a target analyte-binding partner will bind. An analyte oligonucleotide may be from a variety of sources, e.g., human or other mammalian biological fluids or tissues, including blood, urine, cerebrospinal fluid, stool, sputum, or wound exudates, genetic DNA or RNA from bacteria or viruses, food stuffs, environmental materials, etc., and may be prepared for the purification procedure by a variety of means, e.g., proteinase K/SDS, chaotropic salts, or the like. The term "oligonucleotide analyte" is used interchangeably herein with the terms "analyte" and "target analyte."

The term "analyte" as used herein is intended to mean a molecular species to be purified. As noted above, analytes are purified by virtue of their binding to a magnetically responsive particle which has been surface-coated with an analyte-binding partner, to create a particle-binding partner-analyte complex. The complex is thereafter separated from the sample by applying a magnetic field to the sample, thereby immobilizing the analyte against further processing to remove the remaining components of the sample. The term "analyte" is also intended to encompass molecular species which are functionally equivalent to analytes of interest in a particular context. Thus, the term "analyte" includes analyte analogs, analyte fragments, and the like.

The terms "analyte-binding partner," "target-binding partner" and "oligonucleotide-binding partner" as used herein are intended to encompass molecules which will bind to analyte oligonucleotides. Analyte-binding partners may be proteins which recognize general structural features of the target analyte, i.e., they may bind to single-stranded or double-stranded oligonucleotides, or they may recognize specific nucleotide sequences in the target analyte.

The terms "isolation" and "purification" as used herein are intended to mean that a composition containing a specific oligonucleotide analyte, in addition to other materials including other oligonucleotide species, may be significantly enriched in that oligonucleotide, i.e., such that the composition then comprises the oligonucleotide in substantially pure form. Optical or fluorescent methods may be used to measure oligonucleotide purity. See, Schleif et al. (1981) Practical Methods in Molecular Biology, Springer-Verlag, New York. Typically, a "purified" oligonucleotide represents a composition containing at least about 90 wt.%, more particularly at least about 95 wt.%, most particularly at least about 99 wt.%, of that oligonucleotide. In general, a hyperchromicity of 35% at A₂₆₀ is expected for pure DNA. The ratio of A₂₆₀:A₂₈₀ of pure double-stranded DNA is between about 1.65 and 1.85.

Examples of oligonucleotide analyte-binding partners include the IE-2 protein of human cytomegalovirus, which interacts directly with a short nucleotide sequence termed the cis repression signal (Lang et al. (1993) J. Virol. 67:323-331), the *Neisseria gonorrhoeae* DNA-binding protein (Dorward et al. (1989) J. Bacterial 171:4196-4201), filamentous phages M13 and fd gene V products (Alberts et al. (1972) J. Mol. Biol. 68:139-152; Oey et al. (1972) J. Mol. Biol. 68:125-138), phage Ike PIKE protein (Peeters et al. (1983) J. Mol. Biol. 169:197-215), bacteriophage T4 gene 32 protein (Alberts et al. (1970) Nature 227:1313-1318), bacteriophage T7 gene 2.5 protein (Reuben (1973) Proc. Natl. Acad. Sci. USA 70:1846-1850), *E. coli* F sex factor-coded SSF protein (Chase et al. (1983) Proc. Natl. Acad. Sci. USA 80:5480-5484), SSB protein (Molineux (1974) J. Biol. Chem. 249:6090-6098), N4 SSB (Lindberg et al. (1989) J. Biol. Chem. 264:12700-12708, herpes simplex virus type 1 and type 2 major DNA binding protein ICP8 (McGeoch et al. (1988) J. Gen. Virol. 69:1531-1574; Toh et al. (1993) Arch. Virol. 129;183-196) or HSV DNA binding proteins ICP4 and ICSP 11/12 (Lehtinen (1986) Arch. Virol. 87:107-118; Lehtinen et al. (1985) J. Med. Virol. 16:245-256), *S*. *cerevisiae* centromere DNA element I binding protein (Bram et al. (1987) Mol. Cell. Biol. 7:403-409), UV-inducible, damage-specific DNA binding protein (Abrami 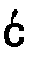 et al. (1991) J. Biol. Chem. 266:22493-22500), MeCP2, a vertebrate DNA binding protein with affinity for methylated DNA (Meehan et al. (1992) Nucleic Acids Res. 200:5085-5092), SAF-A, which has high affinity for nuclear matrix/scaffold attachment DNA (Romig et al. (1992) EMBO J. 11:3431-3440), and the like. Additional proteins which are useful in the claimed invention include DNA polymerase, *lac* repressor and operator, adenovirus major late transcription factor, and Drosophila heat-shock activator protein. Other proteins which bind to single strand DNA with high affinity but without sequence specificity are described by Chase et al. (1986) Ann. Rev. Biochem. 53:103-136.

Additional oligonucleotide-binding partners include RNA-binding proteins such as eukaryotic initiation factor-3, in particular the 62-kDa subunit (Naranda et al. (1994) J. Biol. Chem. 269:32286-32292), human calreticulin, a rubella virus RNA-binding protein (Singh et al. (1994) Proc. Natl. Acad. Sci. USA 91:12770-12774), SecA protein (Kiser et al. (1994) Current Microbiol. 29:323-329), AUF1, which binds to A + U-rich elements (Ehrenman et al. (1994) Gene 149:315-319), RNA-binding proteins K and H16 (Gaillard et al. (1994) Nucleic Acid Res. 22:4183-4186; Aasheim et al. (1994) Nucleic Acids Res. 22:959-964), Tat, an HIV-1 nuclear regulatory protein that binds to *trans*-activation response element RNA (Jones et al. (1994) Ann. Rev. Biochem. 63:717-743), nucleolin, and the like.

In addition, oligonucleotide analyte-binding proteins may be identified using methods well known in the art. Such methods are described, for example, by J.-C. Lelong (1993) Meth. Enzymol. 218:609-618, Khuntirat et al. (1990) J. Virological Meth. 29:97-104, Singh et al. (1987) Biochem. Biophys. Res. Comm. 147:65-70. Furthermore, oligonucleotide-binding proteins may be prepared which have been designed to recognize DNA sequences based on the "Y-shaped scissors grip" model for leucine-zipper gene-regulatory proteins (Park et al. (1992) Proc. Natl. Acad. Sci. USA 89:9094-9096).

In addition, the analyte-binding partner may be an antibody. A target oligonucleotide, or fragments thereof, can be used as an antigen to produce antibodies, either polyclonal, monoclonal, or both, by methods which are well known in the art. If polyclonal antibodies are desired, a selected mammal, (e.g., mouse, rabbit, goat, horse, pig, etc.) is immunized with an desired antigen or a fragment thereof. Serum from the immunized animal is collected and treated according to known procedures. If serum containing polyclonal antibodies is used, the polyclonal antibodies can be purified by immunoaffinity chromatography, using known procedures.

Monoclonal antibodies to a particular oligonucleotide analyte can also be readily produced by one skilled in the art. The general methodology for making monoclonal antibodies by using hybridoma technology is well known. Immortal antibody-producing cell lines can be created by cell fusion, and also by other techniques such as direct transformation of B lymphocytes with oncogenic DNA, or transfection with Epstein-Barr virus. *See, e.g.,* M. Schreier et al., Hybridoma Techniques (1980); Hammerling et al., Monoclonal Antibodies and T-cell Hybridomas (1981); Kennett et al., Monoclonal Antibodies (1980); *see also* U.S. Patent Nos. 4,341,761; 4,399,121; 4,427,783; 4,444,887; 4,452,570; 4,466,917; 4,472,500, 4,491,632; and 4,493,890. Panels of monoclonal antibodies produced against the antigen of interest, or fragment thereof, can be screened for various properties; *i.e*., for isotype, epitope, affinity, etc.

### B. General Methods

In general, the method of this invention involves rapid, easy to perform purification of target oligonucleotides, e.g., DNA, RNA or both, from complex biological samples. Of utmost interest is the isolation and purification of oligonucleotides from relevant blood or tissue samples for clinical diagnosis using hybridization assays. Analysis of purified oligonucleotides is also commonly performed using techniques such as, for example, amplification or sequencing.

The method disclosed herein combines rapid lysis of cellular components in the sample, binding of the released target oligonucleotide to specific oligonucleotide-binding partners linked to magnetically responsive particles and separation of the oligonucleotide from the crude solution by application of a magnetic field.

The use of magnetically responsive particles to effect separation of components from biological samples has been reviewed (Hirschbein et al. (1982) Chemtech March 1982:172-179; Pourfarzaneh (1982) The Ligand Quarterly 5:41-47; and Halling et al. (1980) Enzyme Microb. Techno 2:2-10).

In order to purify oligonucleotides, e.g., DNA, out of a sample which is complex and mixed such as a blood sample, the cellular components in the sample are first subjected to lysing agents such as lysozyme, SDS, Triton X-100, or the like. Agents and methods for lysing cells are well known in the art. See, *for example,* Birnboim (1992) Meth. Enzymol 216:154-160 and Sambrook et al., *supra.*

For example, oligonucleotides can be released from biological systems via a variety of methods including the chemical action of detergents, bases, acids, chaotropes, organics and mixtures of these chemicals on samples. Physical methods of processing samples can be employed and include pressure, heat, freeze-thaw cycles and sonication with or without glass beads. Further, combinations of physical and chemical methods have can be used to prepare samples such as chemical lysis followed by sonication.

Magnetically responsive particles which are surface-coated with oligonuleotide-binding partners are added to a reaction vessel, e.g., a borosilicate glass, polypropylene or polycarbonate test tube, holding a sample containing or suspected of containing a target oligonucleotide analyte to form a suspension. The suspension is incubated for a sufficient duration to allow the surface-coated particles to bind to the desired target, thereby forming a particle-binding partner-analyte complex. The reaction vessel is then placed in close proximity to a source of magnet field to immobilize the complex to the inner surface of the reaction vessel. After decanting, aspirating or otherwise removing the supernatant fluid from the vessel, the particle-binding partner-analyte complex is washed by adding an appropriate buffer or other reagent, removing the vessel from the magnet and resuspending the particle-target complex. The wash procedure may be repeated as often as desired.

Magnetically responsive particles are well known in the art (see U.S. Patent No. 4,672,040 to Josephson) and are commercially available from, for example, Dynal^{®}, Inc. (Lake Success, NY) and Bangs Laboratories, Inc. (Carmel, IN). Magnetically responsive latex particles may be made of divinylbenzene, polystyrene, or other polymers, copolymers, and terpolymers, and have -COOH or -NH₂ surface groups, or other defined chemical functionalities such as aldehyde, aliphatic amine, amide, aromatic amine, haloalkyl, hydrazide or hydroxyl by which biomolecules may be covalently, ionically, adsorptively or otherwise bound. The particles are magnetic, paramagnetic or otherwise responsive to an applied magnetic field. The preferred size of the magnetically responsive particle ranges from 0.5 to 5.0 µ. The particles preferably have an iron oxide content of approximately 10 to 60% by weight and a surface - COOH content of between about 20 to 200 µ equivalents per gram of particles.

The source of the magnetic field may be a permanent magnet, e.g., a ferro- or ferrimagnetic material, or an induced magnet, i.e., an electromagnet. One such magnet is a neodynium-iron-boron permanent magnet (Dynal^{®}, Inc.).

Oligonucleotide analyte-binding partners may be prepared by procedures which have been described in the literature. The binding partners may be purified from biological samples, prepared synthetically from known amino acid sequences, prepared from motifs which have been shown to bind oligonucleotides, e.g., leucine-zipper proteins (Vinson et al. (1989) Science 246:911-916) or Cys₂His₂ zinc-finger proteins (Desjarlais et al. (1993) Proc. Natl. Acad. Sci. USA 90:2256-2260), prepared recombinantly or obtained commercially, e.g., *E. coli* rec A protein (Sigma). In addition, oligonucleotide-binding partners may be isolated using magnetically responsive particles surface-coated with oligonucleotides, or fragments thereof. See, Gabrielsen et al. (1993) Meth. Enzymol. 218:508-525.

The surface of the magnetically responsive particles may be prepared for linking analyte-binding partners thereto by any method well known in the art. One preferred method involves functionalizing the surface prior to coating with a binding partner.

The surface of the magnetically responsive particles is treated with an organosilane coupling agent to functionalize the surface. The organosilane coupling agent is preferably represented by the formula RₙSiY₍₄ₙ) where: Y represents a hydrolyzable group, e.g., alkoxy, typically lower alkoxy, acyloxy, lower acyloxy, amine, halogen, typically chlorine, or the like; R represents a nonhydrolyzable organic radical that possesses a functionality which enables the coupling agent to bond with organic resins and polymers; and n is 1, 2 or 3. One example of such an organosilane coupling agent is 3-glycidoxypropyltrimethoxysilane ("GOPS"), the coupling chemistry of which is well-known in the art. See, for example, Arkins, "Silane Coupling Agent Chemistry," Petrarch Systems Register and Review, Eds. Anderson et al. (1987). Other examples of organosilane coupling agent are (γ-aminopropyl)triethoxysilane and (γ-amino-propyl)trimethoxysilane. Still other suitable coupling agents are well known to those skilled in the art. In the next step, the organosilane coupling agent, now covalently bound to the substrate surface, is derivatized, if necessary, to provide for surface reactive groups which will bind the binding-partner coating. For example, if the organosilane coupling agent provides for surface vicinal diol groups, these can be converted to reactive aldehyde groups by conventional methods (e.g., by reaction with sodium periodate). The reactive aldehyde groups react with the amino groups in protein to form imines (i.e., Schiff bases, - N=C<). Reduction of the imine with a suitable reducing agent such as sodium cyanoborohydride at suitable pH provides the amine derivative and results in the covalent attachment of the protein to the surface of the magnetically responsive particles. Alternatively, if the organosilane coupling agent provides for surface amino groups, these can then react directly with the carboxyl groups present on the protein to form covalent amide bonds. In this embodiment, it may be desirable to activate the carboxyl groups of the protein prior to reaction with the surface amine groups.

Another method of coupling protein to carboxylated- or aminated-latex particles involves incubating the particles and protein in 1-(3-dimethylaminopropyl)-3-ethylcarbodiimide.

Still other methods of binding proteins to substrate surfaces are described in G.T Hermanson et al., "Immobilized Affinity Ligand Techniques," San Diego, CA: Academic Press (1992) at pages 195-197, and S.S. Wong, "Chemistry of Protein Conjugation and Crosslinking," CRC Press (1991), chapter 12. Examples of such other methods include cyanogen bromide and periodate-induced activation of Sepharose, alter which protein can be directly coupled to the activated surface. The surface may also be activated by reaction with tosylchloride to form tosyl-activated particles (Jackson et al. (1990) J. Cell Sci. 96:257-262. Methods of attaching antibodies to magnetically responsive substrates have also been described (*see*, *e.g.,* Kamel et al. (1980) Clin. Chem. 26:1281-1284 and U.S. Patent No. 4,177,253 to Davies et al.) Additional protocols for adsorbing proteins onto particles or chemically linking proteins to particles are described in technical references 13A and 13C, respectively, from Bangs Laboratories.

Magnetically responsive particles to which analyte-binding partners have been linked are then added to the reaction vessel containing the crude sample mixture with the released target analyte in solution. After several minutes incubation time, the analyte-binding partner immobilized to the magnetically responsive particles binds to and captures the target analyte present in the sample solution, thereby forming a particle-binding partner-target analyte complex. The binding partner-linked particle may also be incubated with the sample overnight at 4°C, or under any conditions which would be known to one of skill in the art.

A magnetic field of sufficient strength to attract the magnetically responsive particles, preferably in the range of about 100-1000 Oersteds, is then applied to the outside of the reaction vessel for a sufficiently long period of time to effect the separation of the particle-binding partner-analyte complex from the crude biological mixture. The magnetic force "pulls" the particle-binding partner-analyte complex to the inner wall of the reaction vessel. It will appreciated by one of ordinary skill in the art that the composition of the reaction vessel must be such that it does not interfere with the effect of the externally applied magnetic field on the magnetically responsive particles.

Cellular debris, broken membranes, protein, lipids, etc., are left free in the crude supernatant. This supernatant is easily removed from the reaction vessel by aspiration, decanting, or the like, while keeping the magnet in place.

The particle-binding partner-analyte complex is washed with an appropriate solution to further purify the target oligonucleotide. By withdrawing the magnetic field, the particle-binding partner-analyte complex is released back into suspension. Multiple rounds of application of magnetic field, sample washing and withdrawal of the magnetic field can be applied to achieve the necessary purity of the oligonucleotide.

The target analyte is released from the analyte-binding partner by methods which are well known in the art, e.g., altering the pH of the wash solution, increasing the temperature of the incubation, and the like. A magnetic field can then be applied, thereby attracting the magnetically responsive particles with immobilized analyte-binding partner to the wall of the reaction vessel. The target oligonucleotide is left free in the solution ready for further processing, such as use in diagnostic hybridization assays.

It is to be understood that while the invention has been described in conjunction with the preferred specific embodiments thereof, that the description above as well as the example which follows are intended to illustrate and not limit the scope of the invention. Other aspects, advantages and modifications within the scope of the invention will be apparent to those skilled in the art to which the invention pertains.

The following examples are put forth so as to provide those of ordinary skill in the art with a complete disclosure and description of how to use the method of the invention, and is not intended to limit the scope of what the inventor regards as her invention. Efforts have been made to ensure accuracy with respect to numbers (e.g., amounts, temperature, etc.) but some errors and deviations should be accounted for. Unless indicated otherwise, parts are parts by weight, temperature is in °C and pressure is at or near atmospheric.

### EXAMPLE 1

### PURIFICATION OF NEISSERIA GONORRHOEAE DNA

### A. Covalent attachment of DNA-binding protein to magnetically responsive particles.

Single-stranded-DNA-binding protein (SSB) and RecA protein (both from Unites States Biochemical Corp., Cleveland, OH) are coupled to carboxylated magnetically responsive particles as follows.

Carboxylate-modified magnetically responsive particles (50 mg; Bangs Laboratories) are diluted to 5 ml with 0.1 M 2-(N-morpholino)ethanesulfonic acid (MES) buffer, pH 5.5. The particles are then recovered by application of a magnetic field and the supernatant is discarded. The particles are resuspended in 0.1 M diethanolamine buffer, pH 10.5, containing 45 mg/ml water soluble carbodiimide, e.g., 1-cyclohexyl-3-(2-morpholinoethyl)-carbodiimide, stirred at room temperature for 30 minutes and washed three times with fresh diethanolamine buffer.

The washed particles are resuspended in diethanolamine buffer with 2-7 mg/ml SSB or RecA protein, stirred for 60 minutes at room temperature and washed with buffer. The derivatized particles are stored at 4°C alter saturating them with 1 mg/ml bovine serum albumin.

### B. Purification of Gonococcal DNA-binding proteins.

Gonococcocal whole-cell DNA is purified from cultured *N. gonorrhoeae* 31426 (American Type Culture Collection, Rockville, MD) as described by Dorward et al., *supra.* Ethanol-precipitated DNA is washed once with 70% ethanol and then dissolved at 1 mg/ml in diethanolamine buffer.

SSB or RecA protein-derivatized magnetically responsive particles (~ 5 mg) are added to the DNA solution. The suspension is allowed to incubate for 10-20 minutes and then exposed to a magnetic field by placing a magnet (neodynium-iron-boron permanent magnet, Dynal^{®}, Inc.); the beads are rapidly collected in a firm pellet. After the supernatant is aspirated, the magnet is removed and the pellet is washed with buffer 3 times. The final wash is done with buffer containing 1.0 M NaCl to dissociate the bound DNA from the derivatized beads. The purified DNA is analyzed by agarose gel electrophoresis as described in Sambrook et al., *supra.*

## Claims

1. A method for purifying a target oligonucleotide analyte comprising:
(a) providing a sample containing or suspected of containing the target;
(b) contacting the sample with an oligonucleotide analyte-binding partner, wherein the binding-partner is linked to a magnetically responsive particle;
(c) incubating the sample with the particle, thereby forming a particle-binding partner-analyte complex;
(d) exposing the complex to a magnetic field;
(e) separating the complex from the sample; and
(f) releasing the target analyte from the complex.

2. The method of claim 1, wherein the target oligonucleotide analyte is RNA or DNA.

3. The method of claim 2, wherein the target oligonucleotide is DNA.

4. The method of claim 1, wherein the analyte-binding partner is a protein or an antibody.

5. The method of claim 4, wherein the analyte-binding partner is a protein.

6. The method of claim 5, wherein the protein is a DNA-binding protein.

7. The method of claim 6, wherein the DNA-binding protein is selected from the group consisting of leucine-zipper proteins, Cys₂His₂ zinc-finger proteins, *E. coli* rec A protein, the IE-2 protein of human cytomegalovirus, the *Neisseria gonorrhoeae* DNA-binding protein, filamentous phage M13 gene V product, filamentous phages fd gene V product, phage Ike PIKE protein, bacteriophage T4 gene 32 protein, bacteriophage T7 gene 2.5 protein, *E. coli* F sex factor-coded SSF protein, SSB protein, N4 SSB, herpes simplex virus type 1 major DNA binding protein ICP8, herpes simplex virus type 2 major DNA binding protein ICP8, HSV DNA binding protein ICP4, HSV DNA binding protein ICSP 11/12, *S*. *cerevisiae* centromere DNA element I binding protein, UV-inducible, damage-specific DNA binding protein, MeCP2, SAF-A, DNA polymerase, lac repressor, lac operator, adenovirus major late transcription factor, and Drosophila heat-shock activator protein.

8. The method of claim 2, wherein the target oligonucleotide is RNA.

9. The method of claim 5, wherein the protein is an RNA-binding protein.

10. The method of claim 9, wherein the RNA-binding protein is selected from the group consisting of eukaryotic initiation factor-3, calreticulin, SecA protein, AUF1, RNA-binding protein K, RNA-binding protein H16, Tat, and nucleolin.

11. The method of claim 1, wherein the binding partner is linked to the magnetically responsive particle covalently, ionically or adsorptively.

12. The method of claim 11, wherein the binding partner is linked to the magnetically responsive particle covalently.

13. The method of claim 1, wherein the magnetic field is generated by a permanent or an induced magnet.

14. The method of claim 1, further comprising washing the complex prior to step (f).
